(19) Europäisches Patentamt
European Patent Office
Office européen des brevets

(11) Veröffentlichungsnummer : **0 058 887**
**B1**

(12) # EUROPÄISCHE PATENTSCHRIFT

(45) Veröffentlichungstag der Patentschrift : **11.04.84**

(51) Int. Cl.³ : **A 61 K 9/02**, A 61 K 31/415

(21) Anmeldenummer : **82101001.4**

(22) Anmeldetag : **11.02.82**

(54) **Antimykotische Mittel mit hoher Wirkstoff-Freisetzung in Form von Stiften.**

(30) Priorität : **23.02.81 DE 3106635**

(43) Veröffentlichungstag der Anmeldung : **01.09.82 Patentblatt 82/35**

(45) Bekanntmachung des Hinweises auf die Patenterteilung : **11.04.84 Patentblatt 84/15**

(84) Benannte Vertragsstaaten : **AT BE CH DE FR GB IT LI NL SE**

(56) Entgegenhaltungen :
**DE-A- 2 461 406**
**FR-A- 2 275 194**
**US-A- 3 483 008**
**US-A- 3 502 594**
**CHEMICAL ABSTRACTS, Band 91, No. 21, 19. November 1979 Zusammenfassung 169082t, Seite 120 COLUMBUS, OHIO (US)**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **von Bittera, Miklos**
**Max-Scheler-Strasse 7**
**D-5090 Leverkusen 3 (DE)**.
Erfinder : **Büchel, Karl Heinz, Prof. Dr.**
**Dabringhausener Strasse 42**
**D-5093 Burscheid (DE)**
Erfinder : **Plempel, Manfred, Dr.**
**Pahlkestrasse 5**
**D-5600 Wuppertal 1 (DE)**
Erfinder : **Regel, Erik**
**Bergerheide 72a**
**D-5600 Wuppertal 1 (DE)**

**EP 0 058 887 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Antimykotische Mittel mit hoher Wirkstoff-Freisetzung in Form von Stiften

Die vorliegende Erfindung betrifft neuartige Formulierungen der bekannten antimykotischen Azolderivate, die eine höhere Freisetzung der Wirkstoffe aufweisen und dadurch eine Kurzzeittherapie ermöglichen.

Für die Behandlung von Mykosen der Haut und der Hautanhangsgebilde beim Menschen sind bereits Zubereitungen von antimykotischen Derivaten, z. B. von aus DE-A-2 461 406 bekannten Azolverbindungen bekannt geworden.

FR-A-2 275 194 beschreibt die Verwendung solcher Azolverbindungen in Kosmetika. Mit den genannten antimykotischen Zubereitungen wurden für eine vollständige Sanierung 14 bis 21 Tagen Therapiezeit benötigt.

Um zu einer Verkürzung der Therapiedauer zu kommen, benötigt man, besonders zur Eliminierung der Keime, bzw. um damit eine mykologisch gesicherte Sanierung zu erzielen, eine höhere Freisetzung der Wirkstoffe im wässrigen Milieu. Dafür sind die bekannten Formulierungen nur begrenzt geeignet, weil sich von dem vorhandenen Wirkstoffangebot nur ein kleiner Anteil im Flüssigkeitsvolumen — also am Ort der Infektion — löst. Wenn man nun ohne weitere Erhöhung der Wirkstoffkonzentration eine Verkürzung der Therapiedauer, z. B. auf einen Tag bei einmaliger Applikation, erreichen will, muß man für eine optimale Freisetzung des Wirkstoffes Sorge tragen.

Es wurde nun gefunden, daß Stift-Formulierungen antimykotischer Azolderivate, die neben üblichen Stift-Formulierungshilfsstoffen 3-5 % Benzylalkohol und 2,5-10 % Spreitmittel enthalten, den Wirkstoff in höherem Maße freisetzen und dadurch eine Verkürzung der Therapiedauer auf 1 Tag ermöglichen. Dieser Effekt der höheren Wirkstoff-Freisetzung kann bis zu einer Zehnerpotenz erreichen.

Wirkstoffe, die in dieser Weise formuliert werden können, sind alle antimykotisch wirksamen Azolderivate, insbesondere Imidazol- und Triazolderivate. Sie sind in den erfindungsgemäßen Mitteln in Mengen von 0,05-1 %, vorzugsweise von 0,1-1 % vorhanden.

Beispielsweise seien die Verbindungen der nachstehenden Formeln genannt :

(I)

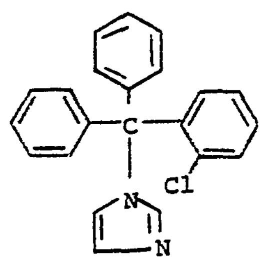

Clotriamzol

(II)

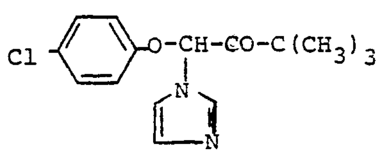

Climbazol

(III)

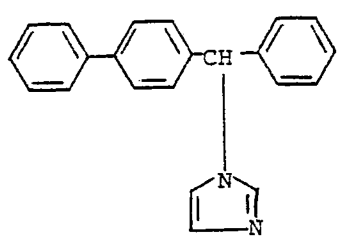

Trifonazol

(IV)

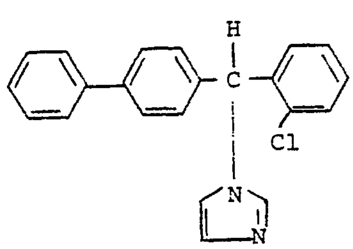

Lombazol

Zahlreiche weitere antimykotisch wirksame Azolderivate sind bekannt aus der DE-OS 2 430 039. Sie können ebenfalls in den erfindungsgemäßen Mitteln als Wirkstoffe dienen.

Unter Spreitmitteln werden ölige Flüssigkeiten verstanden, die sich auf der Haut besonders gut verteilen. [R. Keymer, Pharm. Ind. *32* 1970, 577-581]. Für die erfindungsgemäßen Mittel eignen sich als Spreitmittel insbesondere folgende Verbindungen :

Silikonöle verschiedener Viskosität.

Fettsäureester, wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleyester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandete Estergemische, Myristyllactat, Cetyllactat, 2-Ethylhexylpalmitat, Myristylmyristat u. a.

Triglyceride, wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltiger Fettsäuren, Monoglyceride der $C_8$/$C_{10}$-Fettsäuren u. a.

Fettalkohole, wie Isotridecylalkohol, 2-Octyldodecanol, Cetylstearyl-Alkohol, Oleylalkohol.

Fettsäuren, wie z. B. Ölsäure.

Besonders gut geeignete spreitende Öle sind die folgenden : Isopropylmyristat, Isopropylpalmitat, Capryl/Caprinsäureester von gesättigten Fettalkoholen der Kettenlänge $C_{12}$-$C_{18}$, wachsartige Fettsäureester wie künstliches Entenbürzeldrüsenfett, Silikonöle, Isopropylmyristat/Isopropylstearat/Isopropylpalmitat-Gemisch.

Folgende weitere Hilfs- und/oder Formulierungs-Grundhilfsstoffe können bei der Herstellung der erfindungsgemäßen Mittel verwendet werden : Nichtionogene Emulgatoren hergestellt durch Umsetzung höherer gesättigter Fettalkohole mit Ethylenoxid. Natriumstearat, Cetylstearylalkohol, Stearinsäure, 2-Octyldodecanol (Guerbet-Alkohol), « Glycerylstearate », Mischung von Fettalkoholen, Wachsen und Ölen, Gemisch von Mono- und Diglyceriden der Palmitin- und Stearinsäure, Kokosfettsäuremonoethanolamid, Kolloiddisperses Gemisch aus Cetyl-Stearylalkohol, und Natrium-Cetyl-Stearylsulfat, Ölsäuredecylester, n-Hexadecanol, Laurinsäuremonoethanolamid, Cetylpalmitat, schwach vernetzte Polyacrylsäure von extrem hohem Molekulargewicht.

Glycerin, Paraffin dickflüssig, Paraffin dünnflüssig, Triethanolamin, Collagen, Allantoin, Novantisolsäure, Parfümöle, Natriumhydroxid, Propylenglykol, Di- und Tripropylenglykol, Wachse etc.

Ferner sind geeignet :

a. Substanz, die z. B. eine Suspension stabilisieren können, z. B. Kolloidale Kieselsäure, Montmorillonite u. a.

b. Tenside (beinhaltet Emulgatoren und Netzmittel), z. B.

1. anionaktive, wie Na-Laurylsulfat, Fettalkoholethersulfate, Mono/Dialkylpolyglykoletherorthop hosphorsäureester-Monoethanolaminsalz ;
2. kationaktive, wie Cetyltrimethylammoniumchlorid ;
3. ampholytische, wie Di-Na-N-lauryl-β-ininodipropionat oder Lecithin ;
4. nicht ionogene, z. B. polyoxyethyliertes Rizinusöl, polyoxyethyliertes Sorbitab-Monooleat, Sorbitan-Monostearat, Cetylalkohol, Glycerinmonostearat, Polyoxyethylenstearat, Alkylphenolpolyglykolether.

c. Stabilisatoren zur Verhinderung des bei einigen Wirkstoffen eintretenden chemischen Abbaues wie Antioxydantien, z. B. Tocopherole, Butylhydroxyanisol.

Als Gebildner kommen solche makromolekularen Verbindungen in Frage, die sich sowohl in Wasser als auch in organischen Lösungsmitteln lösen bzw. anquellen können und nach dem Trocknen eine Art Film bilden.

Folgt man einer Einteilung der makromolekularen Hilfsstoffe, [Keipert et al., Die Pharmazie *28* 1973, 145-183] so kommen vor allem ionische Makromoleküle in deren Salzform zur Anwendung. Dies sind unter anderem Natriumcarboxymethylcellulose, Polyacrylsäure, Polymethacrylsäure und deren Salze, Natriumamylopektinsemiglykolat, Alginsäure und Propylenglykol-Alginat als Natriumsalz, arabisches Gummi, Xanthan-Gummi, Guar-Gummi.

Amphotere Makromoleküle wie Protein-Derivate, z. B. Gelatine sind ebenso geeignet wie nichtionische Polymere, z. B. Methylcellulose, Hydroxypropylcellulose und lösliche Stärken, die obige Anforderungen erfüllen.

Als Lösungsmittel sind Wasser und auch alle mit Wasser mischbaren Lösungsmittel geeignet. In Betracht kommen z. B. Alkanole wie Ethanol und Isopropylalkohol, Propylenglykol, Methylcellosolve, Cellosolve, Ester, Morpholine, Dioxan, Dimethylsulfoxid, Dimethylformamid, Tetrahydrofuran, Cyclohexanon.

Es können bei der Herstellung der erfindungsgemäßen Formulierungen ein oder mehrere Lösungsmittel eingesetzt werden.

## Herstellung der Stifte

Die einzelnen Komponenten werden bei 70 °C-75 °C geschmolzen bzw. gelöst. Der Fertigansatz wird entlüftet, indem man die Masse in gelöstem Zustand eine viertel Stunde stehen läßt. Es sollte vermieden

werden, die Schmelze zu lange und höher als notwendig zu erhitzen. Das Ausgiessen der Stifte erfolgt bei ca. 70 °C. Es dürfen sich keine Lufteinschlüsse bilden. Durch Flämmen der Stifte wird eine glänzende Oberfläche erzielt.

Vergleichsbeispiel A : Stift enthält weder Benzylalkohol noch Spreitmittel

| | |
|---|---|
| Trifonazol-Wirkstoff | 1,00 |
| Nichtionogener Emulgator hergestellt durch Umsetzung höherer gesättigter Fettalkohole mit Ethylenoxid | 5,00 |
| Natriumstearat | 8,00 |
| 1,2-Propylenglykol | 15,00 |
| Glycerin wasserfrei | 15,00 |
| Wasser entmineralisiert | 13,00 |
| Ethanol | ad 100,00 |

Vergleichsbeispiel B : Stift enthält Benzylalkohol, jedoch kein Spreitmittel

| | |
|---|---|
| Trifonazol-Wirkstoff | 1,00 |
| Nichtionogener Emulgator hergestellt durch Umsetzung höherer gesättigter Fettalkohole mit Ethylenoxid | 5,00 |
| Natriumstearat | 8,00 |
| 1,2-Propylenglykol | 15,00 |
| Glycerin wasserfrei | 15,00 |
| Wasser entmineralisiert | 10,00 |
| Benzylalkohol | 3,00 |
| Ethanol | ad 100,00 |

Beispiel 1

| | |
|---|---|
| Trifonazol-Wirkstoff | 1,00 |
| Nichtionogener Emulgator hergestellt durch Umsetzung höherer gesättigter Fettalkohole mit Ethylenoxid | 5,00 |
| Natriumstearat | 8,00 |
| 1,2-Propylenglykol | 10,00 |
| Glycerin wasserfrei | 10,00 |
| Wasser | 10,00 |
| Isopropylmyristat | 10,00 |
| Benzylalkohol | 3,00 |
| Parfümöl | 1,00 |
| Ethanol | ad 100,00 |

Beispiel 2

| | |
|---|---|
| Clotrimazol-Wirkstoff | 1,00 |
| Cetylstearylalkohol | 5,00 |
| Natriumstearat | 8,00 |
| 1,2-Propylenglykol | 10,00 |
| Glycerin wasserfrei | 10,00 |
| Wasser | 10,00 |
| Isopropylmyristat | 10,00 |
| Benzylalkohol | 3,00 |
| Parfümöl | 1,00 |
| Ethanol | ad 100,00 |

Beispiel 3

| | |
|---|---|
| Lombazol-Wirkstoff | 1,00 |
| Nichtionogener Emulgator hergestellt durch Umsetzung höherer gesättigter Fettalkohole mit Ethylenoxid | 5,00 |
| Natriumstearat | 8,00 |
| 1,2-Propylenglykol | 10,00 |
| Glycerin wasserfrei | 10,00 |
| Wasser entmineralisiert | 10,00 |
| Isopropylmyristat | 10,00 |

| | |
|---|---|
| Benzylalkohol | 3,00 |
| Parfümöl | 1,00 |
| Ethanol | ad 100,00 |

Beispiel 4

| | |
|---|---|
| Lombazol-Wirkstoff | 0,10 |
| Nichtionogener Emulgator hergestellt durch Umsetzung höherer gesättigter Fettalkohole mit Ethylenoxid | 5,00 |
| Natriumstearat | 8,00 |
| 1,2-Propylenglykol | 10,00 |
| Glycerin wasserfrei | 10,00 |
| Wasser entmineralisiert | 10,00 |
| Isopropylmyristat | 10,00 |
| Benzylalkohol | 3,00 |
| Parfümöl | 1,00 |
| Ethanol | ad 100,00 |

Beispiel 5

| | |
|---|---|
| Trifonazol-Wirkstoff | 1,00 |
| Natriumstearat | 6,00 |
| Wasser entmineralisiert | 5,00 |
| 1,2-Propylenglykol | 22,50 |
| Isopropylmyristat | 2,50 |
| Benzylalkohol | 3,00 |
| Parfümöl | 1,00 |
| Ethanol | ad 100,00 |

Beispiel 6

| | |
|---|---|
| Climbazol-Wirkstoff | 1,00 |
| Nichtionogener Emulgator hergestellt durch Umsetzung höherer gesättigter Fettalkohole mit Ethylenoxid | 5,00 |
| Natriumstearat | 8,00 |
| 1,2-Propylenglykol | 10,00 |
| Glycerin wasserfrei | 10,00 |
| Wasser | 10,00 |
| Isopropylmyristat | 10,00 |
| Benzylalkohol | 3,00 |
| Parfümöl | 1,00 |
| Ethanol | ad 100,00 |

Wirksamkeitstestung der erfindungsgemäßen Mittel am Trichophyton-infiziertem Meerschweinchen

Als Testmodell zur vergleichenden Wirksamkeitsprüfung der erfindungsgemäßen Formulierungen verwendeten wir Trichophyton-infizierte Pirbirgth-white-Meerschweinchen mit einem durchschnittlichen Gewicht von 600 g. Die Tiere wurden auf dem Rücken mit einer elektrischen Haarschneidemaschine so geschoren, daß ca. 1/10 mm lange Haarstümpfe stehen blieben.

Die Infektion mit Trichophyton mentagrophytes erfolgte durch leichtes Verreiben einer 24 Stunden in Sabouraud-Nährlösung angekeimten Sporensuspension des Erregers auf einer ca. 2 × 2 cm großen Fläche des geschorenen Rückens der Tiere. Aufgetragen wurden pro Tier 0,5 ml Keimsuspension, die $1 - 3 \times 10^5$ infektiöse Pilzpartikel enthielten.

Bei diesem Infektionsmodus zeigen sich 2-3 Tage post infectionem die ersten Symptome der Dermatophytose als Rötung und Schuppung der Haut. Bei unbehandelten Tieren ist ca. 14 Tage p. i. die Dermatophytose maximal ausgeprägt : Flächiger Haarausfall und blutige Integument-Defekte innerhalb einer entzündlich veränderten, schuppigen Randzone.

Die zu prüfenden Formulierungen wurden 1-mal, am 2. Tag post infectionem, lokal auf die gerötete Infektionsstelle der Tiere appliziert und mit einem Hornspatel leicht verrieben. Es wurden jeweils 0,5 ml der Formulierung = 5 mg Wirkstoff (1 %ige Formulierung) aufgetragen. Die Bewertung des Infektionsablaufs erfolgte täglich bis zum 20. Tag p. i. Die Testergebnisse sind aus der nachstehenden Tabelle ersichtlich.

| Mittel des Beispiels | Wirkung am Trichophyton-infizierten Meerschweinchen |
|---|---|
| A | ** |
| B | *** |
| 1 | **** |
| 2 | **** |
| 3 | **** |
| 4 | **** |
| 5 | **** |
| 6 | **** |

| | |
|---|---|
| **** | sehr gute Wirkung |
| *** | gute Wirkung |
| ** | Wirkung |
| * | schwache Wirkung |
| 0 | keine Wirkung |

Verwendet man anstelle der erfindungsgemäßen Formulierungen die keine Benzylalkohol und kein Spreitmittel (siehe Vergleichsbeispiele A und B) enthalten, so wird ein Effekt, der demjenigen der erfindungsgemäßen Formulierungen entspricht, erst nach dreimaliger Applikation erreicht.

**Ansprüche** (für die Vertragsstaaten : BE, CH, DE, FR, GB, IT, LI, NL, SE)

1. Antimykotische Mittel mit höherer Freisetzung der Wirkstoffe, enthaltend Azolderivate und übliche Formulierungshilfsstoffe, dadurch gekennzeichnet, daß sie in Stiftform vorliegen und 3-5 % Benzylalkohol und 2,5-10 % Spreitmittel enthalten.

2. Antimykotische Mittel nach Anspruch 1, dadurch gekennzeichnet, daß sie als Wirkstoff Clotrimazol der Formel

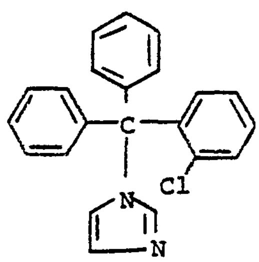

enthalten.

3. Antimykotische Mittel nach Anspruch 1, dadurch gekennzeichnet, daß sie als Wirkstoff die Trifonazol der Formel

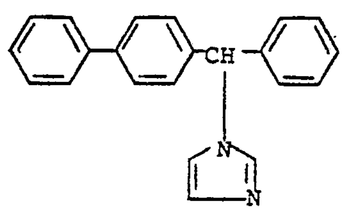

enthalten.

4. Antimykotische Mittel nach Anspruch 1, dadurch gekennzeichnet, daß sie als Wirkstoff Climbazol der Formel

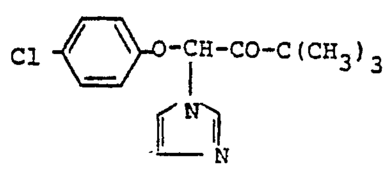

enthalten.

5. Antimykotische Mittel nach Anspruch 1, dadurch gekennzeichnet, daß sie als Wirkstoff Lombazol der Formel

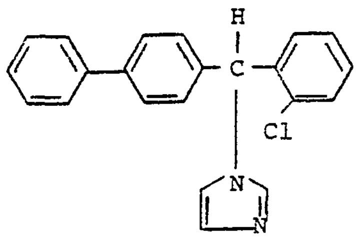

enthalten.

6. Antimykotische Mittel nach Anspruch 1, dadurch gekennzeichnet, daß sie die antimykotischen Azolderivate in Mengen von 0,05-1 %, vorzugsweise von 0,1-1 % enthalten.

**Ansprüche** (für den Vertragsstaat AT)

1. Verfahren zur Herstellung von antimykotischen Stiften, dadurch gekennzeichnet, daß man ein Gemisch aus antimykotischen Azolderivaten, 3-5 % Benzylalkohol, 2,5-10 % Spreitmittel und üblichen Formulierungshilfsmitteln bei 70-75 °C schmilzt, dann entlüftet, in Stiftformen gießt und abkühlen läßt.

2. Verfahren zur Herstellung von antimykotischen Stiften, dadurch gekennzeichnet, daß man ein Gemisch aus Clotrimazol, 3-5 % Benzylalkohol, 2,5-10 % Spreitmittel und üblichen Formulierungshilfsmitteln bei 70-75 °C schmilzt, dann entlüftet, in Stiftformen gießt und abkühlen läßt.

3. Verfahren zur Herstellung von antimykotischen Stiften, dadurch gekennzeichnet, daß man ein Gemisch aus Trifonazol, 3-5 % Benzylalkohol, 2,5-10 % Spreitmittel und üblichen Formulierungshilfsmitteln bei 70-75 °C schmilzt, dann entlüftet, in Stiftformen gießt und abkühlen läßt.

4. Verfahren zur Herstellung von antimykotischen Stiften, dadurch gekennzeichnet, daß man ein Gemisch aus Climbazol, 3-5 % Benzylalkohol, 2,5-10 % Spreitmittel und üblichen Formulierungshilfsmitteln bei 70-75 °C schmilzt, dann entlüftet, in Stiftformen gießt und abkühlen läßt.

5. Verfahren zur Herstellung von antimykotischen Stiften, dadurch gekennzeichnet, daß man ein Gemisch aus Lombazol, 3-5 % Benzylalkohol, 2,5-10 % Spreitmittel und üblichen Formulierungshilfsmitteln bei 70-75 °C schmilzt, dann entlüftet, in Stiftformen gießt und abkühlen läßt.

6. Verfahren zur Herstellung von antimykotischen Stiften, dadurch gekennzeichnet, daß man ein Gemisch aus 0,05-1 %, vorzugsweise 0,1-1 %, antimykotischen Azolderivaten, 3-5 % Benzylalkohol, 2,5-10 % Spreitmittel und üblichen Formulierungshilfsmitteln bei 70-75 °C schmilzt, dann entlüftet, in Stiftformen gießt und abkühlen läßt.

**Claims** (for the Contracting States : BE, CH, DE, FR, GB, IT, LI, NL, SE)

1. Antimycotic agents with higher release of the active compounds, and containing azole derivatives and customary formulation auxiliaries, characterised in that they are in stick form and contain 3-5 % of benzyl alcohol and 2.5-10 % of spreading agent.

2. Antimycotic agents according to Claim 1, characterised in that they contain clotrimazole of the formula

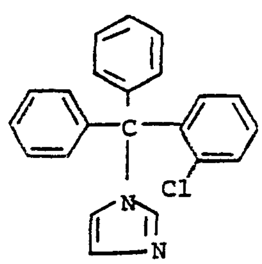

as the active compound.

3. Antimycotic agents according to Claim 1, characterised in that they contain trifonazole of the formula

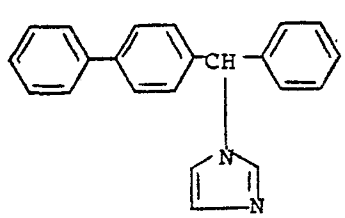

as the active compound.

4. Antimycotic agents according to Claim 1, characterised in that they contain climbazole of the formula

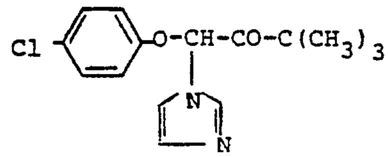

as the active compound.

5. Antimycotic agents according to Claim 1, characterised in that they contain lombazole of the formula

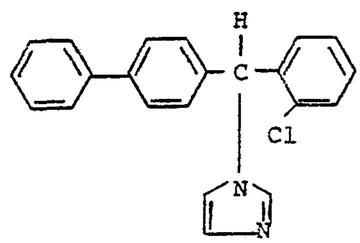

as the active compound.

6. Antimycotic agents according to Claim 1, characterised in that they contain the antimycotic azole derivatives in amounts of 0.05-1 %, preferably of 0.1-1 %.

**Claims** (for the Contracting State AT)

1. Process for the preparation of antimycotic sticks, characterised in that a mixture of antimycotic azole derivatives, 3-5 % of benzyl alcohol, 2.5-10 % of spreading agent and customary formulation auxiliaries is melted at 70-75 °C, and then deaerated, poured into stick moulds and allowed to cool.

2. Process for the preparation of antimycotic sticks, characterised in that a mixture of clotrimazole, 3-5 % of benzyl alcohol, 2.5-10 % of spreading agent and customary formulation auxiliaries is melted at 70-75 °C, and then deaerated, poured into stick moulds and allowed to cool.

3. Process for the preparation of antimycotic sticks, characterised in that a mixture of trifonazole, 3-5 % of benzyl alcohol, 2.5-10 % of spreading agent and customary formulation auxiliaries is melted at 70-75 °C, and then deaerated, poured into stick moulds and allowed to cool.

4. Process for the preparation of antimycotic sticks, characterised in that a mixture of climbazole, 3-5 % of benzyl alcohol, 2.5-10 % of spreading agent and customary formulation auxiliaries is melted at 70-75 °C, and then deaerated, poured into stick moulds and allowed to cool.

5. Process for the preparation of antimycotic sticks, characterised in that a mixture of lombazole, 3-5 % of benzyl alcohol, 2.5-10 % of spreading agent and customary formulation auxiliaries is melted at 70-75 °C, and then deaerated, poured into stick moulds and allowed to cool.

6. Process for the preparation of antimycotic sticks, characterised in that a mixture of 0.05-1 %, preferably 0.1-1 %, of antimycotic azole derivatives, 3-5 % of benzyl alcohol, 2.5-10 % of spreading agent and customary formulation auxiliaries is melted at 70-75 °C, and then deaerated, poured into stick moulds and allowed to cool.

**Revendications** (pour les Etats contractants : BE, CH, DE, FR, GB, IT, LI, NL, SE)

1. Produits antimycotiques à libération accrue des substances actives, contenant des dérivés d'azoles et des produits auxiliaires usuels de formulation, caractérisés en ce qu'ils sont sous forme de bâtons et contiennent de 3 à 5 % d'alcool benzylique et de 2,5 à 10 % d'agents d'étalement.

2. Produits antimycotiques selon la revendication 1, caractérisés en ce qu'ils contiennent en tant que substance active le Clotrimazol de formule

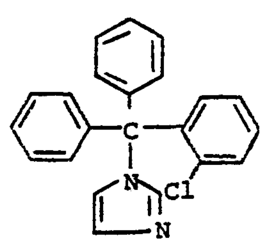

3. Produits antimycotiques selon la revendication 1, caractérisés en ce qu'ils contiennent en tant que substance active le Trifonazol de formule

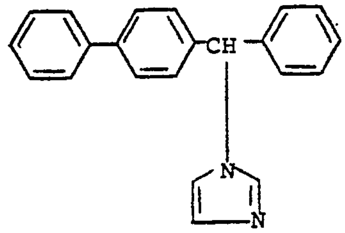

4. Produits antimycotiques selon la revendication 1, caractérisés en ce qu'ils contiennent en tant que substance active le Climbazol de formule

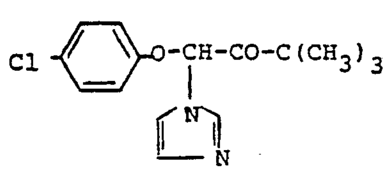

5. Produits antimycotiques selon la revendication 1, caractérisés en ce qu'ils contiennent en tant que substance active le Lombazol de formule

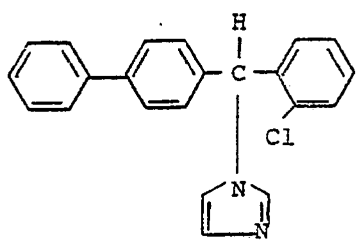

6. Produits antimycotiques selon la revendication 1, caractérisés en ce qu'ils contiennent les dérivés d'azoles antimycotiques en quantités de 0,05 à 1 %, de préférence de 0,1 à 1 %.

**Revendications** (pour l'Etat contractant AT)

1. Procédé pour la fabrication de bâtons antimycotiques, caractérisé en ce que l'on fait fondre à 70-75 °C, un mélange de dérivés d'azoles antimycotiques, 3-5 % d'alcool benzylique, 2,5-10 % d'agent d'étalement et des agents auxiliaires habituels pour formulations, ensuite on désaère, on coule dans des moules en forme de bâton et on fait refroidir.
2. Procédé pour la fabrication de bâtons antimycotiques, caractérisé en ce que l'on fait fondre à 70-75 °C un mélange de Clotrimazol, 3-5 % d'alcool benzylique, 2,5-10 % d'agent d'étalement et des agents auxiliaires habituels pour formulations, ensuite on désaère, on coule dans des moules en forme de bâton et on fait refroidir.
3. Procédé pour la fabrication de bâtons antimycotiques, caractérisé en ce que l'on fait fondre à 70-75 °C un mélange de Trifonazol, 3-5 % d'alcool benzylique, 2,5-10 % d'agent d'étalement et des agents auxiliaires habituels pour formulations, ensuite on désaère, on coule dans des moules en forme de bâton et on fait refroidir.
4. Procédé pour la fabrication de bâtons antimycotiques, caractérisé en ce que l'on fait fondre à 70-75 °C un mélange de Climbazol, 3-5 % d'alcool benzylique, 2,5-10 % d'agent d'étalement et des agents auxiliaires habituels pour formulations, ensuite on désaère, on coule dans des moules en forme de bâton et on fait refroidir.
5. Procédé pour la fabrication de bâtons antimycotiques, caractérisé en ce que l'on fait fondre à 70-75 °C un mélange de Lombazol, 3-5 % d'alcool benzylique, 2,5-10 % d'agent d'étalement et des agents auxiliaires habituels pour formulations, ensuite on désaère, on coule dans des moules en forme de bâton et on fait refroidir.
6. Procédé pour la fabrication de bâtons antimycotiques, caractérisé en ce que l'on fait fondre à 70-75 °C un mélange de 0,05-1 % de préférence 0,1-1 % de dérivés d'azoles antimycotiques 3-5 % d'alcool benzylique, 2,5-10 % d'agent d'étalement et des agents auxiliaires habituels pour formulations, ensuite on désaère, on coule dans des moules en forme de bâton et on fait refroidir.